**Europäisches Patentamt**

**European Patent Office**

**Office européen des brevets**

(19)

(11) Numéro de publication : **0 493 248 A1**

(12) # DEMANDE DE BREVET EUROPEEN

(21) Numéro de dépôt : **91403529.0**

(22) Date de dépôt : **23.12.91**

(51) Int. Cl.⁵ : **B09B 3/00**, C12N 1/20, C12N 1/14, // (C12N1/20;, C12R1:00), (C12N1/14;, C12R1:645)

(30) Priorité : **24.12.90 FR 9016248**

(43) Date de publication de la demande :
**01.07.92 Bulletin 92/27**

(84) Etats contractants désignés :
**AT BE CH DE DK ES FR GB GR IT LI LU NL SE**

(71) Demandeur : **EMC SERVICES**
**62, rue Jeanne d'Arc**
**F-75013 Paris (FR)**

(72) Inventeur : **Bully, François**
**17, rue Sainte Colette**
**F-54505 Vandoeuvre les Nancy (FR)**
Inventeur : **Reisinger, Otto**
**29, Chemin de la Poste, Velaine en Haye**
**F-54840 Gondreville (FR)**

(74) Mandataire : **Desaix, Anne et al**
**Ernest Gutmann - Yves Plasseraud S.A., 67 bld.Haussmann**
**F-75008 Paris (FR)**

(54) **Composition pour la décontamination de sols pollués. Procédé et dispositif pour l'utilisation de cette composition.**

(57)   L'invention concerne une composition comprenant un inoculum à base de cellules procaryotes et/ou eucaryotes notamment de bactéries, de champignons ou de levures ou plusieurs types de ces cellules, sélectionnées pour leur capacité à :

— se développer dans un milieu de culture contenant comme seule source de carbone des contaminants toxiques déterminés, en particulier des contaminants organiques,

— capter, adsorber et accumuler ces contaminants à partir d'un sol à traiter au niveau de leur paroi cellulaire et éventuellement au niveau de leur cytoplasme et/ou par l'intermédiaire de leurs produits de lyse.

L'invention vise aussi l'utilisation de cette composition dans un procédé et un dispositif pour traiter les sols pollués.

EP 0 493 248 A1

Le problème de la dépollution de sols contaminés par des produits chimiques soit accidentellement, soit à la suite de rejets de déchets industriels, est traité selon différentes stratégies. En particulier on connait la possibilité d'utiliser certaines méthodes de traitement physico-chimique et notamment de traiter des quantités déterminées de sols pollués, selon des protocoles d'insinération.

De tels traitements d'incinération présentent des qualités intéressantes mais demeurent néanmoins peu satisfaisants sur le plan de la mise en oeuvre étant donné la nécessité de les réaliser sur des sites appropriés. Ce procédé peut être avantageusement utilisé dans des conditions d'urgence et en cas de forte contamination. Depuis quelques années d'autres tentatives ont été faites pour traiter des sols pollués, notamment en ayant recours à différents microorganismes dont on souhaitait utiliser les capacités de dégrader les agents chimiques toxiques contenus dans des sols pollués.

La mise en oeuvre des capacités que présentent certains microorganismes connus, de dégrader des produits chimiques et notamment des produits chimiques organiques, ne suffit toutefois pas pour le traitement de tous les sols pollués. En effet il est clair que certains types de déchets industriels en particulier d'origine organique ou certaines pollutions accidentelles résistent à un traitement par des microorganismes habituellement utilisés pour leur capacité de biodégradation.

Le problème du traitement des sols contaminés par des produits chimiques résistants aux microorganismes habituellement utilisés pour ce type de traitement restait donc posé.

Il était nécessaire, dans le cadre de la recherche de moyens propres au traitement du sol, de tenir compte de paramètres qui n'entraient pas en jeu dans le cas par exemple du traitement des eaux polluées ou de l'air pollué. En effet le sol constitue de façon générale un substrat hétérogène dans lequel des contaminants sont plus ou moins disponibles en fonction de l'intensité de leur adsorption dans les fractions du sol telles que les argiles ou l'humus.

En général on distingue sur un site pollué le "coeur" de la pollution contenant les teneurs les plus élevées en produits toxiques et la zone du rayonnement de la pollution au niveau de laquelle les teneurs sont plus faibles. Cette zone de rayonnement peut atteindre plusieurs hectares et la profondeur de la pollution une dizaine de mètres ou plus.

Par ailleurs on peut noter que dans le cas d'un traitement d'eau ou d'air, faisant appel à des microorganismes, ces derniers sont adsorbés sur un piège et les contaminants sont amenés au contact de ce piège. Dans le cas du traitement de sol les contaminants sont fixés, notamment au niveau des fractions du sol et les microorganismes sont amenés au contact du sol.

Le passage d'eau dans le sol ne suffit pas à mettre en solution ou en suspension de nombreux contaminants organiques.

S'écartant du principe appliqué dans les techniques connues, selon lequel la dépollution biologique des sols contaminés par des déchets industriels nécessite une dégradation des polluants par des microorganismes, les inventeurs ont mis au point de nouveaux moyens efficaces pour le traitement biologique des sols pollués, sans avoir recours de façon nécessaire à la biodégradation des contaminants.

Pour l'élaboration des moyens selon l'invention, les inventeurs se sont en particulier intéressés au cas de polluants organiques résistants à l'ensemble des microorganismes connus. Des polluants particulièrement résistants à des traitements biologiques courants utilisés dans le cadre de la mise au point des moyens de l'invention, sont des contaminants toxiques organiques appartenant à la famille des polychlorures de biphényle (PCBs), qui appartiennent au groupe des hydrocarbures aromatiques halogénés.

Les résultats obtenus, par mise en oeuvre des moyens de l'invention lors du traitement de PCBs, se sont révélés être applicables pour le traitement d'autres polluants.

A cet égard, les inventeurs ont remarqué de façon tout à fait intéressante et originale, que certains microorganismes peuvent être utilisés pour concentrer des contaminants toxiques sans avoir obligatoirement recours à leur dégradation et par conséquent offrir un moyen d'éliminer les polluants de façon plus aisée qu'en utilisant les méthodes mises en oeuvre à ce jour.

Selon l'invention, les microorganismes utilisables sont capables d'agir comme des biofiltres cellulaires pouvant ainsi capter et accumuler les polluants au moins au niveau de leur paroi cellulaire.

Cette action des microorganismes de l'invention a lieu tout au long de leur parcours dans le sol traité et est notamment favorisée par la circulation ou la recirculation simultanée d'un flux liquide constitué par exemple par l'eau de lixiviation traitée si besoin.

L'invention propose par conséquent des compositions à base de microorganismes, susceptibles d'intervenir dans le traitement de sols contaminés par des contaminants toxiques.

L'expression "contaminants toxiques" s'applique dans le cadre de l'invention pour désigner des produits chimiques organiques ou minéraux, toxiques ou dangereux de par leur nature et/ou leur concentration et qui doivent être éliminés du sol. De façon particulièrement avantageuse, l'invention propose des moyens adaptés au traitement des contaminants organiques toxiques, notamment des contaminants de la famille des PCBs.

L'invention concerne également un procédé pour le traitement de sols pollués par des contaminants toxiques, ainsi qu'un dispositif pour la mise en oeuvre de ce procédé.

Les moyens de l'invention ont la propriété tout à fait intéressante de favoriser la bioimmobilisation et/ou la bioaccumulation des contaminants organiques présents dans le sol, ainsi que le cas échéant leur biodégradation.

De plus, ils offrent l'avantage de permettre l'élimination de traces de contaminants.

Une composition selon l'invention est caractérisée en ce qu'elle est destinée à la décontamination d'un sol pollué par un ou plusieurs contaminants toxiques déterminés et ce qu'elle comprend un inoculum à base de cellules procaryotes et/ou eucaryotes notamment de bactéries, de champignons ou de levures ou de plusieurs types de ces cellules, sélectionnées pour leur capacité à :

– se développer dans un milieu de culture contenant comme seule source de carbone des contaminants toxiques déterminés, en particulier des contaminants organiques,

– capter, adsorber et accumuler ces contaminants à partir du sol à traiter, au niveau de leur paroi cellulaire et éventuellement au niveau de leur cytoplasme et/ou par l'intermédiaire de leurs produits de lyse.

Différentes mesures réglementent la protection de l'environnement, notamment au niveau des sols. Ainsi actuellement les sols contenant plus de 100 ppm de PCBs doivent être traités par une installation agréée. Pour une teneur comprise entre 10 et 100 ppm, les terres sont mises en décharge de classe 1 (classification établie par les administrations concernées). La tendance générale de l'évolution de la législation en matière de normes, conduit à envisager une baisse de ce seuil maximum à 50 ppm dans le futur. Par la mise en oeuvre des moyens de décontamination conformes à l'invention on peut atteindre un niveau de teneur inférieure à 50 ppm. De façon générale, les moyens de l'invention permettent de répondre et de s'adapter aux exigences de la réglementation en matière de traitement de la pollution.

Un avantage des compositions de l'invention est qu'elles permettent l'élimination, au moins partielle, de traces de polluants.

Selon un premier mode de réalisation avantageux de l'invention, une composition préférée répondant à la définition donnée ci-dessus, est en outre caractérisée en ce qu'elle est sensiblement dépourvue de cellules ne se développant pas en présence d'une source de carbone constituée exclusivement par les susdits contaminants toxiques.

La capacité des cellules contenues dans l'inoculum, de se développer lorsqu'elles sont mises en culture dans un milieu dont la seule source de carbone est constituée par un ou plusieurs contaminants toxiques déterminés, peut être vérifiée par la mise en oeuvre d'un test tel que celui décrit dans les exemples, qui permet de déterminer la résistance des souches au produit toxique à éliminer.

D'une façon générale, un tel test comprend :

a) l'ensemencement d'un milieu de culture minéral adapté pour le développement d'organismes cellulaires du type procaryotes et/ou eucaryotes, ce milieu contenant en outre à titre de seule source de carbone, au moins un contaminant toxique déterminé, avec une quantité déterminée de cellules eucaryotes ou procaryotes choisies,

b) l'incubation à une température donnée, de préférence la température optimale de croissance des souches eucaryotes ou procaryotes par exemple entre 4°C et 35°C environ de préférence entre 10°C et 35°C, notamment supérieure à 15°C, de façon particulièrement avantageuse entre environ 20 et environ 32°C, de façon particulièrement avantageuse environ 30°C pour des bactéries et environ 25°C pour des microorganismes filamenteux ou des levures pendant un temps suffisant pour favoriser le développement des cellules résistantes aux contaminants,

c) l'étalement sur un milieu nutritif spécifique des différents types cellulaires présents pour estimer leur capacité de développement,

d) le cas échéant un ou plusieurs repiquages successifs des cultures avec des concentrations croissantes de contaminants et la répétition de l'étape c) après chaque repiquage,

e) la culture avec un milieu nutritif exempt d'extrait de levure, spécifique des différents types cellulaires présents pour estimer leur capacité de développement,

f) la récupération des souches capables de se développer dans ce milieu.

L'absence d'extrait de levure est destinée à vérifier que le développement des souches cellulaires présentes est effectivement dû à la présence des contaminants. L'extrait de levure suffit en effet à certaines souches cellulaires pour leur développement.

Les compositions selon l'invention, en particulier lorsqu'elles contiennent des cellules vivantes (les parois des cellules mortes peuvent néanmoins parfois conserver leur activité) peuvent, en plus de la bioimmobilisation et de la bioaccumulation, permettre une biométabolisation des contaminants, dont l'efficacité peut être vérifiée à l'aide de différents paramètres et par exemple par les suivants :

– par diminution de la teneur en PCBs. Dans ce cas il est nécessaire de s'assurer que cette baisse n'est pas provoquée par des facteurs non biologiques (adsorption non spécifique sur les cellules ou sur la verrerie) qui sont parfois difficilement appréciables rigoureusement. La modification du profil chromatographique est la seule preuve irréfutable du métabolisme ;

– par superposition des chromatogrammes (témoin et essai) et calcul du pourcentage de dégradation de chaque congénère (molécule spécifique d'une famille) ou groupe de congénères représenté par un pic. Les hauteurs de pics ou les surfaces peuvent être prises en compte. Cette méthode nécessite des conditions d'extraction et d'analyse identiques, notamment la concentration en PCBs dans l'extrait. Elle devient complexe lorsqu'il s'agit de comparer plus de 2 chromatogrammes ;

– par calcul d'un ratio de surface de pic. Cette méthode contourne le problème de la reproductibilité des conditions analytiques et permet de suivre aisément une évolution dans le temps. Puisqu'il est connu que les congénères les plus chlorés sont peu ou non biodégradables, ils ne subissent pas ou peu de modification de teneur. Leurs surfaces sur des chromatogrammes, avec des conditions opératoires similaires, restent identiques. Un pic parmi les derniers est choisi comme pic de référence. Les pics importants du chromatogramme peuvent être repérés avec le logiciel Apex (Stang. Intruments). Ce repérage reste identique dans le temps. Les ratios de surface de pics sont obtenus en divisant la surface de chaque pic repéré par celle du pic de référence. Au cours d'une expérimentation, une diminution du ratio d'un pic signifie une baisse de la surface de ce pic par rapport à celle du pic de référence. En conséquence, cette méthode permet de repérer un pic correspondant à un congénère métabolisable. Cette interprétation n'est valable que si le pic de référence n'est pas modifié. Elle permet de s'affranchir des variations de teneurs dans les extraits.

En outre, le pic de référence doit bien être individualisé en fin de chromatogramme. Il possède une surface individuelle suffisamment importante afin que l'incertitude liée à l'intégration reste minime. Une variation de ratio est significative si elle est supérieure ou égale à 10%.

Le repèrage des pics et le choix du pic de référence varient en fonction des expérimentations. Cependant cette méthode originale d'interprétation connaît une limite : un pic représentant un congénère peut régresser par rapport à sa valeur initiale sans que cela n'apparaisse significativement par comparaison des ratios.

De façon remarquable les inventeurs ont en outre fourni une autre technique pour vérifier l'efficacité d'un inoculum dans le cadre de la mise en oeuvre de l'invention : ils ont montré que l'efficacité d'un inoculum de l'invention peut être vérifiée par l'observation de caractéristiques ultrastructurales des cellules de l'inoculum, au microscope électronique à transmission (M.E.T.). L'observation au M.E.T., des cellules d'un inoculum ayant préalablement été mis en contact avec un échantillon contaminé par des polluants organiques, en particulier des contaminants de la famille des PCBs, permet de façon inattendue, de constater la présence de granules denses, correspondant à ces contaminants, à l'intérieur des cellules de l'inoculum.

En d'autres termes, les inventeurs ont démontré que de façon surprenante des molécules non hydrosolubles telles que celles de PCBs, sont capables de pénétrer dans des cellules procaryotes ou eucaryotes.

Ces molécules sont principalement localisées à la périphérie des cellules, au niveau de leurs parois mais elles peuvent également être présentes dans le cytoplasme, voire à proximité du noyau cellulaire.

Ainsi, la mise en évidence de la possibilité de détecter des granules de contaminants organiques, notamment de PCBs, fournit les moyens d'un test pour vérifier l'efficacité d'un inoculum donné, dans le cadre de l'application à la décontamination d'un sol pollué. Cette technique constitue un moyen de sélection des cellules les plus intéressantes dans le cadre de l'invention.

Une composition adaptée à la réalisation de l'invention comprend par exemple des champignons notamment filamenteux en particulier de la famille des Ascomycètes, par exemple Fusarium, Aspergillus, ou Penicillium. On peut utiliser également des champignons unicellulaires comme des levures dans l'inoculum.

Une telle composition comprend aussi avantageusement des bactéries telles que celles décrites dans les exemples.

Les inventeurs ont mis en évidence que l'action de l'inoculum sur les contaminants toxiques peut être modulée, selon qu'il contient des bactéries ou des cellules eucaryotes. Par exemple les bactéries captent les contaminants, par l'intermédiaire de leurs produits de lyse. Les cellules eucaryotes et/ou procaryotes sélectionnées selon la méthodologie décrite, sont capables de capter, d'adsorber et d'assimiler les contaminants au niveau de leur paroi, voire de leur cytoplasme.

Selon une autre définition, une composition satisfaisante pour la réalisation de l'invention est caractérisée par les propriétés suivantes :

– elle est destinée à la décontamination d'un sol pollué par un ou plusieurs contaminants toxiques déterminés,

– elle est obtenue à partir des souches cellulaires naturellement présentes dans un sol contaminé, par sélection par exemple en mettant en oeuvre les étapes suivantes :

a) l'ensemencement d'un milieu de culture minéral adapté pour le développement d'organismes cellulaires du type procaryotes et/ou eucaryotes, ce milieu contenant en outre à titre de seule source de carbone, au moins un contaminant toxique déterminé, avec une quantité déterminée de cellules eucaryotes ou procaryotes choisies,

b) l'incubation à une température donnée, de préférence la température optimale de croissance des souches eucaryotes ou procaryotes par exemple entre 4°C et 35°C environ de préférence entre 10°C et 35°C, notamment supérieure à 15°C, de façon particulièrement avantageuse entre environ 20 et environ 32°C, de façon particulièrement avantageuse environ 30°C pour des bactéries et environ 25°C pour des microorganismes filamenteux ou des levures pendant un temps suffisant pour favoriser le développement des cellules résistantes aux contaminants,

c) l'étalement sur un milieu nutritif spécifique des différents types cellulaires présents pour estimer leur capacité de développement,

d) le cas échéant un ou plusieurs repiquages successifs des cultures avec des concentrations croissantes de contaminants et la répétition de l'étape c) après chaque repiquage,

e) la culture avec un milieu nutritif exempt d'extrait de levure, spécifique des différents types cellulaires présents pour estimer leur capacité de développement,

f) la récupération des souches capables de se développer dans ce milieu.

Avantageusement une composition obtenue par la méthode ci-dessus décrite peut subir une étape supplémentaire de sélection consistant à rechercher en microscopie électronique à transmission la présence de granules denses correspondant à des polluants, incorporés dans les cellules, en particulier au niveau des membranes cellulaires.

Selon un mode de réalisation préféré de l'invention, la composition est caractérisée en ce que l'inoculum comprend des cellules capables de résister et de se développer en culture avec des contaminants de la famille des PCBs, de les capter, de les adsorber, et de les accumuler au niveau de leur paroi cellulaire, voire au niveau de leur cytoplasme.

Selon un autre mode de réalisation avantageux de l'invention, la composition comprend des microorganismes dont la lyse libère des produits captants selon la définition précédemment donnée.

La famille des PCBs comporte de très nombreuses molécules (congénères) présentant un taux de substitution en particulier par le chlore, variable. La très grande diversité de cette famille, conduit à des résultats très différents lorsque l'on recherche des moyens pour traiter des sols contaminés par les PCBs. L'invention est de façon avantageuse, adaptée au traitement de sols contaminés par tous les types de PCBs et notamment par des PCBs appartenant aux isomères les plus stables de cette famille. A titre d'exemple, on mentionne que l'invention est particulièrement adaptée au traitement des PCBs connus sous les marques Aroclor 1260 et Aroclor 1242, qui ont une stabilité différente.

En d'autres termes la composition selon l'invention peut être particulièrement adaptée au traitement de sols contaminés par des PCBs hautement substitués (congénères les plus chlorés).

La composition selon l'invention constitue un filtre biologique, utilisable pour la lixiviation à travers un sol chargé en matière chimique toxique, en particulier en produits d'origine organique.

La composition selon l'invention permet avantageusement de décontaminer des sols pollués, quel que soit le niveau de profondeur des contaminants toxiques.

Les pyralènes ou leur dérivés ne sont pas les seuls constituants d'origine organique susceptibles d'être traités au moyen de la composition selon l'invention.

En effet d'autres compositions selon l'invention sont encore caractérisées en ce que les cellules de l'inoculum sont choisies pour leur capacité à résister et à se développer en culture avec des contaminants toxiques halogénés ou non halogénés, possédant le cas échéant un ou plusieurs cycles aromatiques en particulier des composés tels que les huiles lourdes, des pesticides, des fongicides ou des insecticides ou des solvants organiques et de capter, d'adsorber et d'accumuler ces contaminants organiques toxiques au niveau de leur paroi cellulaire.

De même l'invention fournit des moyens pour traiter des sols contaminés par des polluants non organiques, en particulier par des métaux lourds.

Dans certains cas on peut avantageusement utiliser pour l'élaboration de la composition selon l'invention, des cellules susceptibles de métaboliser, au moins partiellement, les contaminants organiques toxiques. Par exemple l'inoculum peut comprendre des cellules capables par exemple de transformer certains PCBs en des isomères moins stables.

De façon avantageuse, l'inoculum de la composition comprend des cellules permettant ou favorisant la désorption des contaminants organiques toxiques associés à certaines fractions du sol.

A titre d'exemple il peut être intéressant de réaliser une désorption des contaminants, notamment des PCBs, liés à des fractions argileuses du sol.

Les cellules comprises dans la composition peuvent consister uniquement en des cellules vivantes physiologiquement actives ou au contraire en des cellules vivantes en mélange avec des cellules physiologiquement inactives de même origine ou non, ayant conservé leur capacité de captation, d'adsorption et d'accumulation des contaminants.

Lorsque la composition comprend un mélange de cellules vivantes et de cellules mortes ayant conservé les propriétés ci-dessus, les proportions respectives de chaque catégorie sont déterminées en fonction de l'utilisation faite et éventuellement du stade auquel l'inoculum est utilisé pour traiter le sol contaminé. En effet on peut avoir intérêt à mettre en oeuvre une proportion plus élevée de cellules physiologiquement inactives lorsqu'une seconde administration de l'inoculum est opérée.

Un exemple d'inoculum particulièrement avantageux pour réaliser l'invention comprend les souches de bactéries dont la description est donnée dans les exemples et qui sont désignées par Xlbp1, Xlbp3, Xlbp4, Xlbp6, Xlnb2, Xlnb5, Xlc1.

D'autres inoculums préférés contiennent une ou plusieurs des bactéries ci-dessus et/ou une ou plusieurs cellules eucaryotes, par exemple choisies parmi les champignons.

D'autres éléments peuvent naturellement être incorporés dans une composition de l'invention. A cet égard on peut citer des nutriments, capables de stimuler la croissance des cellules de l'inoculum et/ou leur activité dans le sol. On peut mentionner à titre d'exemple des minéraux, par exemple apportés sous la forme de sulfates. On ajoutera par exemple de l'azote, du phosphore, du potassium, du fer, du zinc, du cuivre, du magnésium, du soufre, du cobalt, du molybdène ou tout autre oligoélément.

La stimulation de la croissance des cellules peut en effet faciliter et/ou accélérer leur activité vis à vis des contaminants organiques toxiques.

D'autres éléments pouvant intervenir dans l'élaboration des compositions sont par exemple des agents tensio-actifs qui peuvent être soit produits par des souches cellulaires ayant de telles propriétés ou encore des substances chimiques habituellement utilisées en tant que tensio-actif.

L'apport d'agents tensio-actifs est déterminé en fonction de la nature du sol traité et l'on peut citer à titre d'exemple que cet apport peut être particulièrement intéressant dans le cas d'un sol argileux ou dans le cas d'un sol en général riche en matière humique, matière organique vivante et matière organique morte.

De même un agent tensio-actif peut constituer une nouvelle source de carbone, différente de celle fournie par le contaminant, et générer un effet bénéfique pour la croissance des microorganismes de l'inoculum. De plus la présence de tensio-actifs peut améliorer la désorption des contaminants organiques adsorbés sur certaines fractions du sol.

Etant donné l'importance dans les moyens de l'invention, de la captation et de la fixation par les parois cellulaires des souches de l'inoculum, des différents contaminants du sol, on peut avantageusement mettre en oeuvre des cellules dont les parois auront été épaissies ou dont la zone mucilagineuse externe a été épaissie, ou encore dont la surface pariétale est accrue.

S'agissant de l'accroissement de la paroi pariétale, on peut avoir recours à des cellules mortes qui permettent néanmoins la fixation des contaminants et en particulier des PCBs tant au niveau de la surface externe que de la surface interne de la paroi.

Pour épaissir les parois mucilagineuses des cellules de l'inoculum, on peut mettre en oeuvre les techniques classiques.

En deuxième phase du traitement du sol contaminé, on peut avoir intérêt à favoriser l'activité de prédateurs des cellules, notamment celles de l'inoculum, pour faciliter la séparation, plus spécialement la lyse ou la destruction partielle des parois des cellules ayant adsorbé les contaminants du sol, des autres cellules. Ces prédateurs sont généralement naturellement présents dans le sol traité.

Ils peuvent aussi être ajoutés à la composition comprenant l'inoculum ci-dessus.

On peut également favoriser l'action de prédateurs de certaines populations cellulaires de l'inoculum, lorsque l'on cherche à augmenter la surface pariétale disponible pour l'adsorption des contaminants.

Un prédateur intéressant pour la mise en oeuvre de l'invention appartient à la famille des thécamoebiens bactériophages ou mycophages. En particulier dans une composition selon l'invention utilisable pour la décontamination de sols contenant des PCBs, un prédateur avantageux est l'amibe T Granifera.

La composition de l'invention peut être conservée sous forme d'une solution comprenant l'inoculum sous forme de cellules en suspension.

D'autres modes de conservation de l'inoculum sont néanmoins adaptés à la réalisation de l'invention; on peut citer à titre d'exemple la conservation de l'inoculum sous une forme lyophilisée. De manière générale on peut envisager de conserver la composition de l'invention sous toutes formes adaptées à la conservation des souches cellulaires.

Les compositions selon l'invention sous l'une des formes de conservation choisies peuvent contenir, des agents tensio-actifs chimiques ou des microorganismes susceptibles de produire ces agents. On peut leur ajouter également une ou plusieurs sources de nutriments minéraux (N,P, K, Ca, oligoéléments ...), une source de carbone ou des résidus industriels contenant du matériel biologique capable d'entrer dans la composition de l'inoculum.

L'invention vise naturellement l'utilisation des compositions précédemment décrites pour le traitement de sols contaminés par des polluants organiques toxiques et en particulier par des polluants de la famille des PCBs.

L'invention vise également un procédé de traitement de sols contaminés par des polluants organiques toxiques, caractérisé en ce qu'il comprend l'administration à travers une quantité de sols donnée d'une composition telle que décrite précédemment, dans des conditions permettant la lixiviation de cette composition à travers le sol et, la récupération dans l'eau de lixiviation des cellules ayant accumulé les contaminants toxiques.

Un procédé de traitement avantageux selon l'invention est caractérisé en ce qu'il comprend :

– l'épandage et/ou l'injection dans le sol d'une composition répondant aux définitions ci-dessus, de façon à permettre la lixiviation de cette composition à travers le sol, dans des conditions telles que les contaminants toxiques du sol sont captés par les cellules de l'inoculum, adsorbés au niveau de leur paroi cellulaire et éventuellement de leur cytoplasme, ou encore dans des conditions telles que les contaminants toxiques sont captés par l'intermédiaire des produits de lyse (par exemple d'autolyse) des cellules de l'inoculum, ces contaminants étant le cas échéant métabolisés et l'inoculum agissant éventuellement après désorption à partir de certaines fractions du sol,

– la récupération de l'eau de lixiviation contenant notamment les cellules ayant adsorbé les contaminants toxiques,

– le traitement de l'eau de lixiviation pour séparer les cellules ayant accumulé les contaminants toxiques,

– le cas échéant la répétition des étapes précédentes, à partir de l'eau de lixiviation traitée.

Dans certains cas on aura avantage à utiliser à nouveau l'eau de lixiviation après traitement, pour des raisons économiques mais aussi pour éviter d'introduire des polluants dans le sol, par l'eau de lixiviation rejetée.

On peut selon une variante de l'invention effectuer plusieurs administrations de l'inoculum au cours du traitement, par exemple au stade de l'administration à travers le sol dans la phase initiale de mise en oeuvre du procédé, ou encore au stade de la récupération de l'eau de lixiviation, avant le traitement de cette dernière.

Selon une autre variante de réalisation du procédé de l'invention, l'inoculum administré soit à plusieurs étapes de réalisation du procédé ou seulement à l'étape initiale contient des cellules physiologiquement inactives.

La récupération des contaminants organiques accumulés par les cellules de l'inoculum est réalisée par floculation du matériel cellulaire présent dans l'eau de lixiviation et/ou par coagulation des débris cellulaires ou encore par dégradation des cellules par un prédateur, par exemple une amibe telle que T Granifera, dégradation suivie d'une récupération des PCBs.

Le procédé selon l'invention peut être mis en oeuvre soit sous forme de traitement in situ, soit après excavation du sol.

De même on pourra dans certains modes de réalisation de l'invention, épandre sur le sol à traiter, avec l'eau de lixiviation, des composés permettant d'augmenter l'épaisseur de la paroi, et/ou l'épaisseur de la zone mucilagineuse externe des cellules de l'inoculum et des cellules autochtones.

L'invention vise par ailleurs un dispositif pour mettre un oeuvre le procédé ci-dessus décrit, caractérisé en ce qu'il comprend :

– des moyens pour réaliser l'épandage et/ou l'injection d'une composition répondant aux définitions précédentes et permettre la lixiviation de cette solution à travers la terre à traiter,

– des moyens pour recueillir et le cas échéant traiter l'eau de lixiviation.

D'autres avantages et caractéristiques de l'invention apparaissent dans les exemples qui suivent et dans les figures.

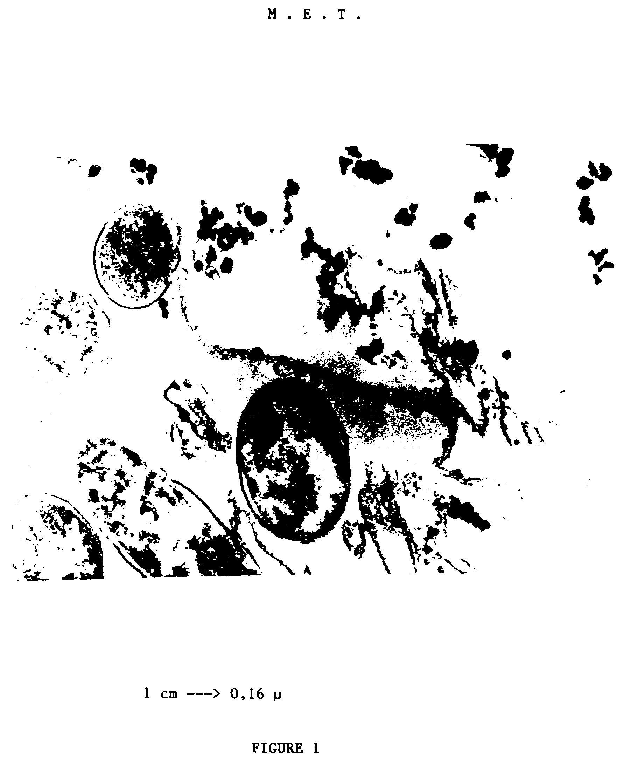

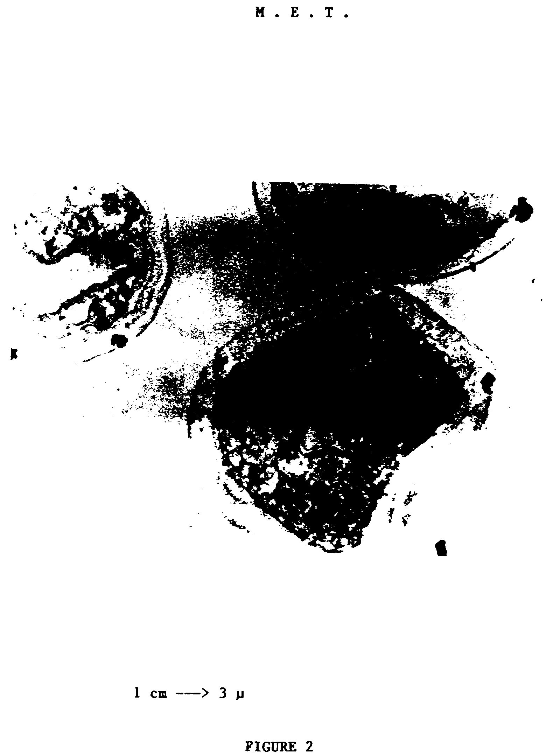

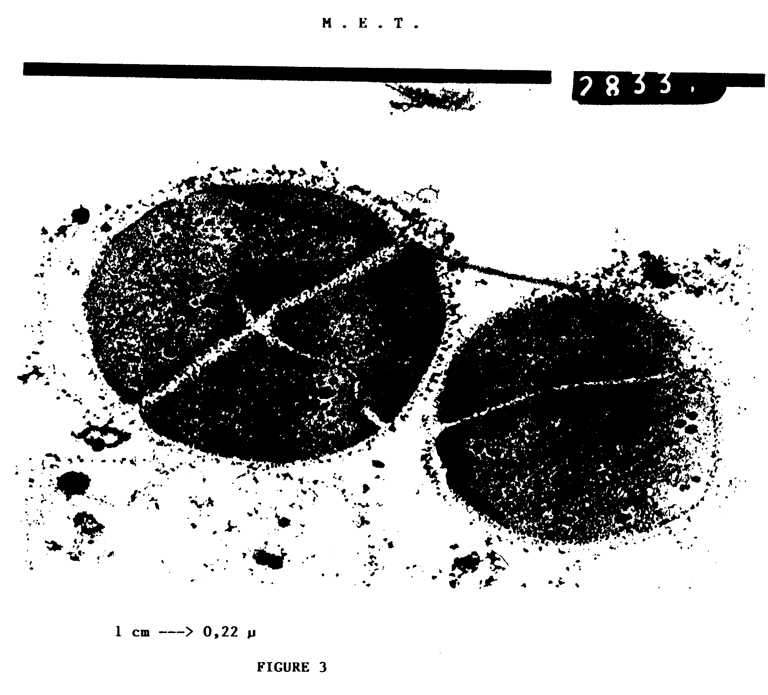

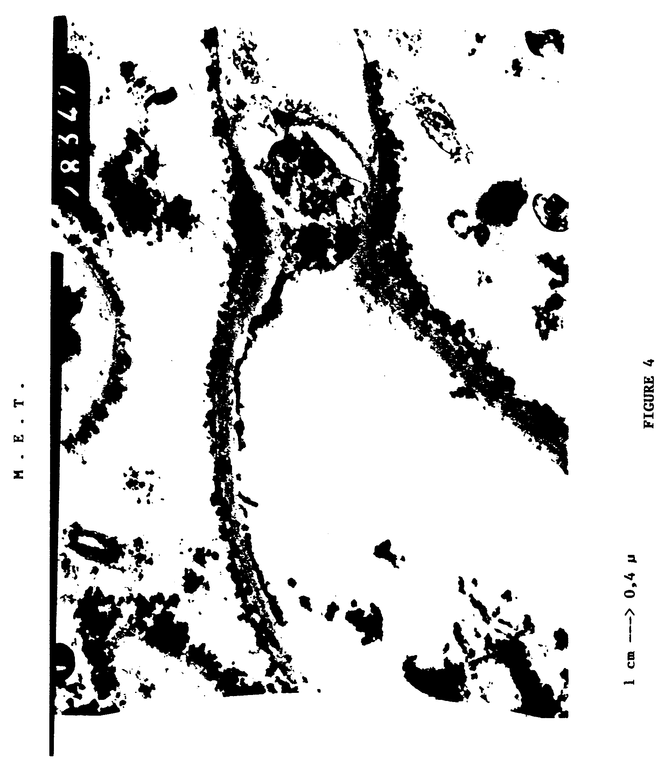

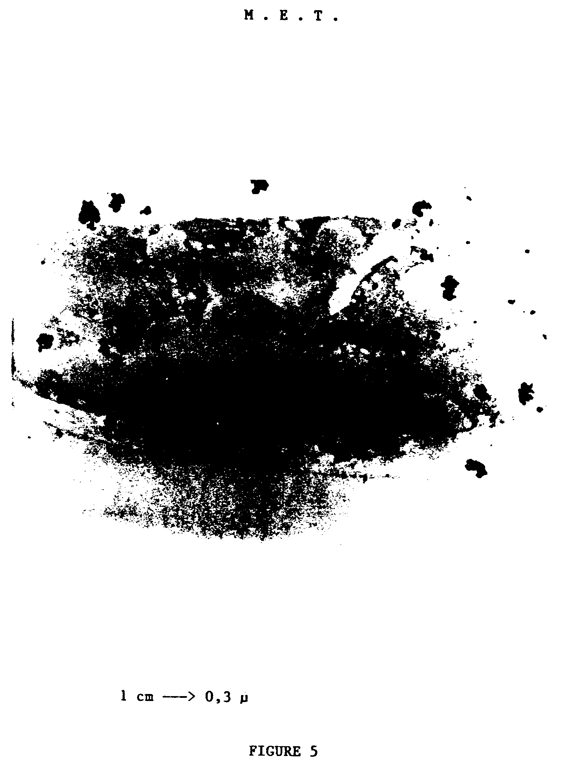

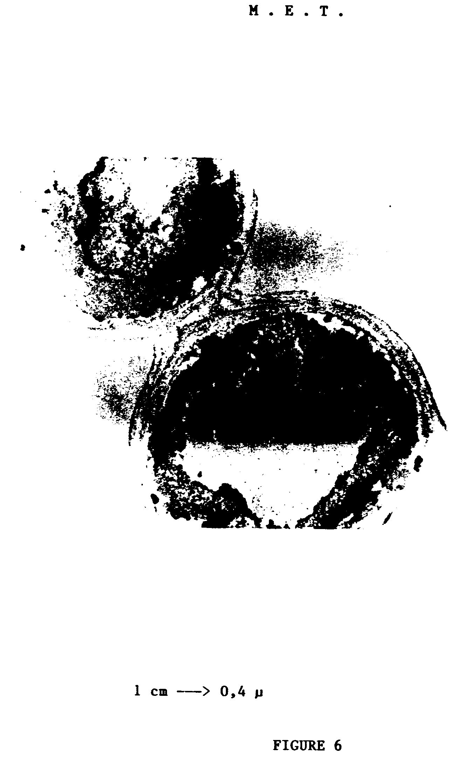

- <u>Figures 1 à 6</u> :

Ces figures présentent des photos obtenues par étude ultrastructurale de coupes ultrafines, au microscope électronique à transmission (M.E.T.). Elles montrent la présence sur les échantillons testés, d'argiles, filaments fongiques, levures, bactéries, mucilages.

Les images obtenues révèlent des corpuscules inconnus jusqu'ici dans des sols contaminés. Ces granules denses aux électrons possèdent un contour diffus et se localisent habituellement aux périphéries des microagrégats. Il sont significativement plus nombreux dans les zones mucilagineuses externes des cellules microbiennes. La double affinité des corpuscules pour la périphérie des microagrégats d'une part, et pour la zone mucilagineuse externe des germes liée à leur nature diffuse, d'autre part, sont des caractéristiques non signalées pour un quelconque constituant des sols. Ces granules représentent des substances liées à la présence de l'Aroclor 1260.

L'observation au M.E.T. confirme que les granules denses aux électrons n'existent que dans les cultures contenant des PCBs (photos 1 à 6). En présence de PCBs, la présence de granules est non seulement relevée mais on remarque aussi une stucture fibrillaire et granuleuse interne. Cette différence structurale concerne essentiellement l'organisation structurale de la cellule en présence de l'Aroclor 1260.

- Figure 7 :

Description des étapes effectuées pour l'obtention des souches.

## I - EXEMPLE D'OBTENTION D'UN INOCULUM

Les souches que l'on souhaite sélectionner pour la préparation des inoculum sont obtenues à partir de milieux naturels contaminés depuis plusieurs années par des produits classés selon la législation comme toxiques ou dangereux. Ces souches ont déjà subi une sélection dans leur biotope initial. Des échantillons de ces milieux ont été utilisés pour la préparation des inoculum.

S'il s'agit d'obtenir un inoculum efficace pour le traitement de sols contaminés par un produit déterminé, un milieu naturel contaminé depuis plusieurs années par ce produit est utilisé comme échantillon de départ.

## ETAPES PRELIMINAIRES

Avant la sélection des souches on met en oeuvre les étapes préliminaires suivantes :
– recherche et dosage d'un contaminant X déterminé dans un échantillon représentatif du milieu naturel défini ci-dessus,
– essais de solubilisation du contaminant X en vue de la mise au point de la technique de sélection,
– dénombrement total de la flore microbienne présente dans un échantillon de milieu naturel choisi, suivant la technique de la suspension-dilution détaillée ci-après :

Dénombrement, technique de la suspension-dilution

Définition :

Le dénombrement total correspond à l'ensemble des microorganismes se développant à 25/30°C. Il s'agit à la fois de bactéries, levures, Actinomycètes et filamenteux.

Le dénombrement sur milieu NB (Bouillon Nutritif) correspond aux bactéries et Actinomycètes.

Le dénombrement sur milieu MG (Extrait de Malt) correspond aux levures et filamenteux.

Les résultats sont exprimés en UFC (Unités Formant des Colonies) par gramme d'échantillon.

Méthodologie :

On stérilise un erlen contenant 100 ml d'eau physiologique stérile (NaCl à 9%), on pèse 10 g de milieu naturel constituant l'échantillon que l'on introduit sous conditions stériles dans l'erlen.

On soumet cet échantillon dans l'erlen à une agitation orbitale durant 1 heure.

A partir de la solution de l'erlen, on prépare des dilutions décimales avec transfert de 1 ml dans 9 ml d'eau stérile. Puis on étale sur gélose 0,1 ml de chaque dilution, en commençant par la plus élevée.

Composition des milieux gélosés utilisés, par litre :

* pour le dénombrement des bactéries et actinomycètes :
15 g de bouillon nutritif (NB)
15 g d'agar
ajustement de pH à 7-7,5, avec une solution de soude, s'il y a lieu.

stérilisation

* pour le dénombrement des levures et filamenteux :
15 g d'extrait de malt (MG)
15 g d'agar
ajustement de pH à 7-7,5, avec une solution de soude, s'il y a lieu.

stérilisation

réajustement du pH à 4-5, avec une solution d'acide phosphorique afin d'inhiber la croissance des bactéries, s'il y a lieu.

Après l'étalement sur milieu gélosé, on réalise une incubation à 30°C, pour les cultures sur NB, durant 48 à 72 heures et à 25°C, pour les cultures sur MG, durant 3 à 4 jours.

On procède ensuite au comptage des unités formant des colonies sur la gélose qui en comprend entre 30 et 300.

## SELECTION DES SOUCHES

A partir d'une suspension réalisée lors de l'étape préliminaire du dénombrement, des milieux de culture liquides contenant le contaminant toxique X déterminé comme seule source de carbone sont ensemencés. Après cette première phase, plusieurs repiquages successifs sont réalisés afin d'éliminer la probabilité de croissance à partir d'éléments nutritifs propres à des cellules physiologiquement mortes, et apportées avec l'ensemencement, et d'affirmer la qualité des souches par augmentation régulière de la teneur en contaminants toxiques.

En parallèle, une sélection est éventuellement réalisée dans les mêmes conditions, le contaminant toxique X étant substitué par un produit de structure chimique proche ou intéressante (exemple : le biphényle dans le cas où le contaminant correspond à des PCBs).

Le détail de cette technique est présenté ci-après :

TECHNIQUE DE LA SELECTION DES SOUCHES

Composition des milieux de sélection

– milieu minéral pour la sélection de bactéries, spécifique aux PCBs.
1,6 g $KH_2PO_4$
4,4 g $K_2HPO_4$
2,1 g $NH_4Cl$
0,4 g $MgSO_4$, $7H_2O$
0,05g $MnSO_4$, $H_2O$
0,01g $FeSO_4$, $7H_2O$
0,003g $CaCl_2$, $2H_2O$
complétés à 1 litre avec de l'eau distillée ou permutée.

Stérilisation

– milieu minéral pour la sélection de levures et filamenteux
2,0 g $NaNO_3$
0,5 g $MgSO_4$, $7H_2O$
0,5 g KCl
0,01g $FeSO_4$, $7H_2O$
complétés à 980 ml d'eau permutée ou distillée.
stérilisation
à froid, on ajoute les solutions stériles suivantes :
10 ml de $KH_2PO_4$ à 10%
5 ml de $ZnSO_4$, $7H_2O$ à 0,2%
5 ml de $CuSO_4$, $5H_2O$ à 0,1%
– milieu minéral pour la sélection de bactéries, actives vis à vis d'autres produits organiques que les PCBs
20 ml de solution A
10 ml de solution B
10 ml de solution C
0,1ml de solution D
2 ml de solution E
complétés à 1 litre avec de l'eau distillée ou permutée

Stérilisation

Solution A :     34 g de $KH_2PO_4$

             44,5g de $Na_2HPO_4, 2H_2O$

complétés à 1 litre avec de l'eau distillée ou permutée

Solution B :     35 g de $MgSO_4, 7H_2O$

complétés à 1 litre avec de l'eau distillée ou permutée

Solution C :     100 g de $NO_3NH_4$

complétés à 1 litre avec de l'eau distillée ou permutée

Solution D :     50 mg de $CuSO_4, 5H_2O$

             100 mg de $H_3BO_3$

             100 mg de $MnSO_4, H_2O$

             100 mg de $ZnSO_4, 7H_2O$

             100 mg de $Na_2MoO_4, 2H_2O$

             100 mg de $CoCl_2, 6H_2O$

complétés à 1 litre avec de l'eau distillée ou permutée

Solution E :     10 g de $CaCl_2$

             200 mg de $FeSO_4$

complétés à 1 litre avec de l'eau distillée ou permutée.

Méthodologie :

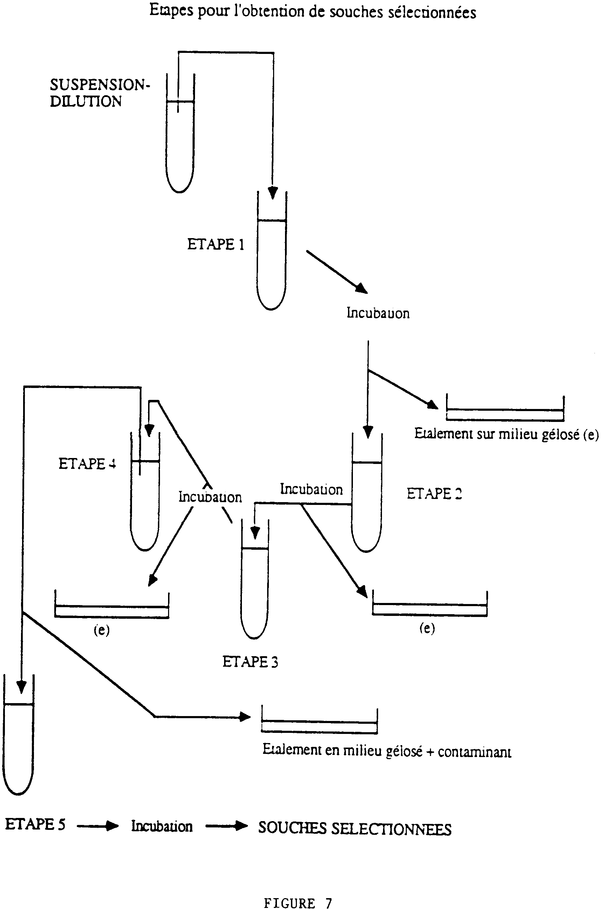

On procède à l'ensemencement des cultures en milieu liquide avec une teneur (a) en contaminant toxique X déterminé, à partir des dilutions décimales $10^{-2}$ et $10^{-3}$ pour les bactéries de $10^{-1}$ et $10^{-2}$ pour les levures et champignons. Ensuite on fait incuber à 30 °C pour la sélection de bactéries et à 25°C pour la sélection de filamenteux et levures, durant 20 jours (étape 1, figure 7).

Puis on réalise un étalement sur milieux gélosés suivi d'une incubation et d'une estimation du développement. Ensuite on procède à des repiquages successifs de cultures en milieux liquides avec des teneurs (b) (c) (d) en contaminant respectivement pour les étapes 2, 3 et 4, avec entre chaque étape une phase d'étalement sur milieux gélosés. Ces ensemencements sont réalisés à partir de la culture de l'étape précédente.

   – valeurs a, b, c et d en cultures liquides :

| Contaminants toxiques X | a | b | c | d |
|---|---|---|---|---|
| Aroclor 1242 | 0,01 | 0,1 | 0,5 | 1,0 |
| Aroclor 1260 | 0,01 | 0,1 | 0,5 | 1,0 |
| Biphényle | 0,20 | 2,0 | 2,0 | 2,0 |
| Toluène | 1,0 | 2,0 | 2,0 | 5,0 |
| Xylène | 1,0 | 2,0 | 2,0 | 5,0 |
| Pyridine | 1,0 | 2,0 | 2,0 | 5,0 |
| Acrylonitrile | 1,0 | 1,0 | 2,0 | 2,0 |
| Perchloréthylène | 1,0 | 1,0 | 2,0 | 2,0 |
| Benzène | 1,0 | 1,0 | 2,0 | 5,0 |
| Hexanone 2 | 1,0 | 1,0 | 2,0 | 5,0 |
| Ester d'acétate | 1,0 | 1,0 | 2,0 | 5,0 |

Les teneurs dans le tableau sont exprimées en pourcentage massique pour les trois premiers produits et en pourcentage volumique pour les suivants.

   – Remarque : lors des étapes 1, 2, 3 et 4, on est en présence d'extrait de levure.

A l'issue de l'étape 4, on procède aux cultures suivantes :

   * cultures sur milieux liquides de même composition que précédemment, à l'exception de l'extrait de levure. Il s'agit de l'étape 5.

   * cultures sur milieux gélosés, soit NB et Agar additionné de contaminant X (sans extrait de levure), en parallèle pour les bactéries et MG pour les levures et filamenteux

le milieu Agar auquel on a ajouté le contaminant déterminé X, est obtenu avec un mélange de 35 g d'Agar et de 15 g de contaminant déterminé pour 100 g de gélose, de manière à obtenir une pâte homogène.

Au moment de couler les plaques, les tubes de milieu minéral gélosé sont réchauffés. Après refroidissement jusqu'à la température de 50°C, 1 g du mélange gélose purifiée-contaminant est incorporé dans chaque tube. Le tout est homogénéisé sur un agitateur rotatif pour tube (Vortex), et la plaque est coulée. Elle doit se solidifier presque immédiatement.

## PREPARATION D'INOCULUM

A l'issue de la sélection des souches, des séries de cultures mixtes sont obtenues; elles peuvent être rassemblées notamment lorsque les cultures sur milieu solide indiquent la présence d'un faible nombre de souches.

La technique de préparation des inoculum, dans laquelle la source de carbone est alors un sucre est décrite ci-après. Le saccharose est choisi pour la facilité de mise en oeuvre. D'autres sucres par exemple le lactose, le glucose peuvent le remplacer.

TECHNIQUE DE PREPARATION DES INOCULUM

Pour 1, 5 litre de milieu propre aux bactéries, on utilise 100 ml de solution F, 100 ml de solution G, 1285 ml d'eau distillée ou permutée et on procède à une stérilisation avant d'ajouter à froid, 15 ml de solution H. Les compositions des solutions F, G, H sont données ci-après.

On ensemence à partir d'une culture liquide issue de l'étape 5 puis on incube en fermenteur ou sur agitateur à mouvement orbital pendant 48 heures.

De préférence, à l'échelle industrielle le fermentateur est équipé d'une agitation mécanique, l'axe d'agitation possédant des turbines du type RUSHTON et le réacteur comporte des contre-pales. Il s'agit lors de cette étape, d'éviter la formation de pelotes (pellets) et l'agrégation des cellules, afin de ne pas diminuer le nombre de sites accessibles.

Solution F :     66 g de $K_2HPO_4$
               25,5 g de $KH_2PO_4$
               32,1 g de $NH_4Cl$
complétés à 1 litre avec de l'eau distillée ou permutée

Solution G :     150 g de saccharose
               15 g d'extrait de levure
complétés à 1 litre avec de l'eau distillée ou permutée

Solution H :     3 9, 4 9 g de $MgSO_4, 7H_2O$
               5,0 g de $MnSO_4, H_2O$
               1,0 g de $FeSO_4, 7H_2O$
               0,3 g de $CaCl_2, 2H_2O$
complétés à 1 litre avec de l'eau distillée ou permutée et avec addition de 5 ml de $H_2SO_4$ concentré.

Pour 1,5 litre de milieu propre aux levures et filamenteux on utilise 100 ml de solution I, 100 ml de solution J de composition décrite ci-après, 1300 ml d'eau distillée ou permutée et on procède à une stérilisation avant d'ajouter à froid des solutions, stérilisées individuellement, suivantes :
        2,5 ml de $CuSO_4, 5H_2O$ à 0,3%
        2,5 ml de $ZnSO_4, 5H_2O$ à 0,6%
        10,0 ml de $KH_2PO_4$ à 15%

Solution I :     7,5 g de KCl
               30,0 g de $NaNO_3$
               7,5 g de $MgSO_4, 7H_2O$
               0,15g de $FeSO_4, 7H_2O$
complétés à 1 litre avec de l'eau distillée ou permutée

Solution J :     150 g de saccharose
               15 g d'extrait de levure
complétés à 1 litre avec de l'eau distillée ou permutée.

## II - CONSERVATION DES INOCULUM ET DES SOUCHES ISOLEES

Milieux cryoprotecteurs :
– pour les souches bactériennes sélectionnées sur les PCBs commercialisés sous les marques Aroclor 1242, Aroclor 1260 et pour le biphényle :

150 g de DMSO

1,5 g de NB

complétés à 1 litre avec de l'eau saturée et Aroclor 1242

stérilisation

– pour les autres souches bactériennes :

150 g de DMSO

1,5 g de NB

complétés à 1 litre avec de l'eau distillée ou permutée

stérilisation

– pour les levures et filamenteux sélectionnés sur Aroclor 1242, Aroclor 1260 et biphényle :

solution de glycérol à 108, préparée avec de l'eau distillée ou permutée

stérilisation

Méthodologie :

– 0,15 ml de matériel biologique est mis en suspension dans 0,75 ml de milieu cryoprotecteur :

Le matériel biologique est :

– le milieu de culture en fin de l'étape 5 de la sélection ou

– une culture sur NB liquide de 48 heures, dans le cas de bactéries ou

– une culture sur MG liquide de 5 jours, dans le cas de levures et filamenteux.

Les récipients utilisés sont des cônes Eppendorf.

Puis on effectue une congélation à - 80°C.

Les cônes sont placés au moins pendant 30 minutes dans une boite en polystyrène, elle-même installée dans le congélateur.

La décongélation a lieu au bain-marie à 37°C

La remise en culture est réalisée comme suit : 0,5 ml du contenu du cône décongelé est rapidement repris dans un milieu NB (pour les bactéries) ou un milieu MG (pour les levures et filamenteux) liquide, et un milieu gélosé NB ou MG est ensemencé en stries.

## III CARACTERISATION DES SOUCHES BACTERIENNES

Les souches bactériennes sont caractérisées en fonction de leur aspect morphologique et de leurs propriétés biochimiques après isolement, selon les techniques microbiologiques connues. Elles font notamment appel à l'utilisation des galeries Api (marque déposée) 20 E et Zym et font l'objet des tests suivants. Pour mémoire on rappelle que les dénominations utilisées s'agissant des caractères mis en évidence sur les galeries API 20E et APIZYM sont les suivantes :

EP 0 493 248 A1

| SOUCHE : | | API 20E | APIZYM |
|---|---|---|---|
| | | ONPG | 1 |
| | | ADH | 2 |
| ORIGINE : | | LDC | 3 |
| | | ODC | 4 |
| | | CIT | 5 |
| MORPHOLOGIE : | | H2S | 6 |
| | | URE | 7 |
| * colonie : forme - hauteur - | | | |
| marge - teinte - pigmentation | | TDA | 8 |
| | | IND | 9 |
| | | VP | 10 |
| * état frais : forme - mobilité | | GEL | 11 |
| | | GLU | 12 |
| * Gram - spore | | MAN | 13 |
| | | INO | 14 |
| PHYSIOLOGIE : | | SOR | 15 |
| | | RHA | 16 |
| * type respiratoire | | SAC | 17 |
| * oxydase | | MEL | 18 |
| * catalase | | AMY | 19 |
| * nitrate réductase | | ARA | 20 |
| * type métabolique | | MAL | |
| | | FRU | |
| | | GAL | |

CULTURE SUR POLLUANT

Les caractères sont présentés par inoculum.

Le nom de la souche correspond à un code interne.

La culture sur le polluant en milieu solide (bp : développement sur biphényle, c : pyralène de condensateur) est précisée par les signes :

　　　 + : bon développement

　　　 ± : développement moyen

　　　 - : faible développement

Les caractères négatifs des galeries API 20E apparaissent sous la forme d'un signe négatif. L'intensité de la coloration d'un caractère positif sur les galeries APIZYM est notée de 1 à 5.

EP 0 493 248 A1

Abréviations :

| API 20E | | | APIZYM |
|---------|--|--|--------|

ONPG:bêta-galactosidase  1 : Témoin coloration culture
ADH :arginine dihydrolase  2 : Phosphatase alcaline
LDC :lysine décarboxylase  3 : Estérase
ODC :ornithine décarboxylase 4 : Estérase Lipase
CIT :utilisation du citrate  5 : Lipase
H2S :production d'$H_2$S  6 : Leucine arylamidase
URE :uréase  7 : Valine arylamidase
TDA :tryptophane désaminase  8 : Cystine arylamidase
IND :production d'indole  9 : Trypsine
VP  :production d'acétoïne 10 : α Chymotrypsine
GEL :gélatinase  11 : Phosphatase acide
GLU :glucose      (*)  12 : Naphtol-AS-BI-phosphohydrolase
MAN :mannitol     (*)  13 : α Galactosidase
INO :inositol     (*)  14 : β Galactosidase
SOR :sorbitol     (*)  15 : β Glucuronidase
RHA :rhammose     (*)  16 : α Glucosidase
SAC :saccharose   (*)  17 : β Glucosidase
MEL :mélibiose    (*)  18 : N-acétyl-β-glucosaminidase
AMY :amydaline    (*)  19 : α Mannosidase
ARA :arabinose    (*)  20 : α Fucosidase
MAL :maltose      (a)
FRU :fructose     (a)
GAL :galactose    (a)

(*): Fermentation/oxydation
(a): Activité/inactivité

- <u>Composition des inoculums utilisés</u>

Les souches de ces inoculums sont strictement aérobies. L'inoculum IV (souches eucaryotes) est constitué par un mélange de Fusarium tabacinum et de différentes levures (notamment une levure "rose")

L'inoculum XI est constitué par les souches procaryotes suivantes obtenues selon la méthodologie décrite ci-après :

– Isolement (selon les techniques connues) de chaque souche constituant la culture mixte à l'issue de la dernière étape de sélection selon la description donnée dans les pages précédentes.

Quatre milieux solides de cultures sont ensemencés à partir d'une suspension de la culture mixte :

– NB,
– agar + biphényle,
– agar + Aroclor 1242,
– agar + Aroclor 1260.

Les caractères morphologiques et biochimiques étudiés sont décrits ci-dessus.

Certaines souches isolées se révèlent identiques. Les caractères obligatoires pour définir une similitude entre des souches sont :
- le gram (+ ou -),
- la mobilité (oui ou non),
- l'aspect de la colonie,
- la forme de la colonie,
- la sporulation (oui ou non), pour les souches gram + seulement,
- l'oxydase (+ ou -).

En outre, 60% de similitude doivent être atteints avec l'ensemble des autres caractères, à l'exception des caractères APIZYM.

Lorsqu'il subsiste un doute, ces derniers permettent de le lever.

| SOUCHE : | | API 20E | APIZYM | |
|---|---|---|---|---|
| XIbp1 | | ONPG- | 1 | |
| | | ADH + | 2 | 1 |
| ORIGINE : | | LDC +/- | 3 | 2 |
| Site contaminé par des PCBs | | ODC +/- | 4 | 3 |
| | | CIT + | 5 | 1 |
| MORPHOLOGIE : | | H2S - | 6 | 4 |
| | | URE +/- | 7 | 1 |
| * colonie : forme - hauteur - | | | | |
| marge - teinte - pigmentation | TDA - | | 8 | |
| bombée, marge irrégulière, crème, | IND - | | 9 | |
| circulaire | VP  - | | 10 | |
| * état frais : forme - mobilité | GEL - | | 11 | 1 |
| bacille, mobile | GLU - | | 12 | 1 |
| * Gram - spore | MAN - | | 13 | |
| GRAM - | INO - | | 14 | |
| PHYSIOLOGIE : | | SOR - | 15 | |
| | | RHA - | 16 | |
| * type respiratoire:aérobie strict | SAC - | | 17 | |
| * oxydase: + | MEL + | | 18 | |
| * catalase: + | AMY - | | 19 | |
| * nitrate réductase: NO3 | ARA - | | 20 | |
| * type métabolique: inactif | | | | |

CULTURE SUR POLLUANT

bp +/-

SOUCHE :

XIbp3

ORIGINE :

Site contaminé par des PCBs

MORPHOLOGIE :

* colonie : forme - hauteur - marge - teinte - pigmentation bombée, marge droite, crème, grasse, circulaire
* état frais : forme - mobilité bacille, mobile
* Gram - spore

GRAM -

PHYSIOLOGIE :

* type respiratoire:aérobie strict
* oxydase: +
* catalase: +
* nitrate réductase: NO2
* type métabolique: oxydatif

| | API 20E | APIZYM | |
|------|---------|--------|---|
| ONPG- | | 1 | |
| ADH - | | 2 | 1 |
| LDC - | | 3 | 1 |
| ODC - | | 4 | 1 |
| CIT - | | 5 | |
| H2S - | | 6 | 2 |
| URE + | | 7 | 1 |
| TDA - | | 8 | |
| IND - | | 9 | 1 |
| VP - | | 10 | |
| GEL - | | 11 | 1 |
| GLU - | | 12 | |
| MAN - | | 13 | |
| INO - | | 14 | |
| SOR - | | 15 | |
| RHA - | | 16 | |
| SAC - | | 17 | |
| MEL - | | 18 | |
| AMY - | | 19 | |
| ARA - | | 20 | |

CULTURE SUR POLLUANT

bp +/-

SOUCHE :

XIbp4

ORIGINE :
Site contaminé par les PCBs

MORPHOLOGIE :

* colonie : forme - hauteur -
  marge - teinte - pigmentation
plate, marge droite, translucide
circulaire
* état frais : forme - mobilité
bacille, mobile
* Gram - spore
GRAM -

PHYSIOLOGIE :

* type respiratoire:aérobie strict
* oxydase: +
* catalase: +
* nitrate réductase: NO3
* type métabolique: oxydatif

| | API 20E | APIZYM |
|---|---|---|
| ONPG- | 1 | |
| ADH + | 2 | 3 |
| LDC +/- | 3 | |
| ODC - | 4 | 3 |
| CIT +/- | 5 | 1 |
| H2S - | 6 | 2 |
| URE + | 7 | 1 |
| TDA - | 8 | 1 |
| IND - | 9 | |
| VP  + | 10 | |
| GEL - | 11 | 1 |
| GLU - | 12 | 1 |
| MAN - | 13 | |
| INO - | 14 | |
| SOR - | 15 | |
| RHA - | 16 | |
| SAC - | 17 | |
| MEL + | 18 | |
| AMY - | 19 | |
| ARA - | 20 | |

CULTURE SUR POLLUANT

bp +++

| SOUCHE : | API 20E | APIZYM | |
|----------|---------|--------|---|
| XIbp6 | ONPG- | 1 | |
| | ADH + | 2 | 4 |
| ORIGINE : | LDC + | 3 | 1 |
| Site contaminé par les PCBs | ODC + | 4 | |
| | CIT + | 5 | |
| MORPHOLOGIE : | H2S - | 6 | 2 |
| | URE + | 7 | |
| * colonie : forme - hauteur - | | | |
| marge - teinte - pigmentation | TDA - | 8 | |
| plate, marge droite, crème- | IND - | 9 | |
| jaunâtre, circulaire, | VP + | 10 | |
| * état frais : forme - mobilité | GEL - | 11 | 1 |
| bacille, mobile | GLU - | 12 | 1 |
| * Gram - spore | MAN - | 13 | |
| GRAM - | INO - | 14 | |
| PHYSIOLOGIE : | SOR - | 15 | |
| | RHA - | 16 | |
| * type respiratoire:aérobie strict | SAC - | 17 | |
| * oxydase: + | MEL - | 18 | |
| * catalase: + | AMY - | 19 | |
| * nitrate réductase: NO2 | ARA - | 20 | |
| * type métabolique: alcalinisation | | | |

CULTURE SUR POLLUANT

bp +++

SOUCHE :                                    API 20E    APIZYM

XInb2                                       ONPG+         1

                                            ADH  -        2      5


ORIGINE :                                   LDC  +        3      1

Site contaminé par les PCBs                 ODC  -        4      4

                                            CIT  +        5      1


MORPHOLOGIE :                               H2S  -        6      1

                                            URE  -        7      1

* colonie : forme - hauteur -

  marge - teinte - pigmentation             TDA  -        8      1

plate, marge sinueuse, jaune                IND  -        9      1

translucide, circulaire,                    VP   -       10      1

* état frais : forme - mobilité             GEL  +       11      2

bacille, mobile                                  GLU  -                12

2

* Gram - spore                             MAN  -       13

GRAM -                                      INO  -       14


PHYSIOLOGIE :                              SOR  -       15

                                           RHA  -       16

* type respiratoire:aérobie strict SAC  -       17      2

* oxydase: -                               MEL  -       18

* catalase: -                              AMY  -       19

* nitrate réductase: NO3                   ARA  -       20

* type métabolique: alcalinisation

à 24 heures, puis inactivité       _____


                                           CULTURE SUR POLLUANT

                                                  c:   -

                                                  bp:  -

| SOUCHE : | | API 20E | | APIZYM |
|---|---|---|---|---|
| XIc1 | | ONPG- | 1 | |
| | | ADH + | 2 | 1 |
| ORIGINE : | | LDC + | 3 | 1 |
| Site contaminé par les PCBs | | ODC +/- | 4 | 3 |
| | | CIT + | 5 | |
| MORPHOLOGIE : | | H2S - | 6 | 2 |
| | | URE +/- | 7 | 1 |
| * colonie : forme - hauteur - | | | | |
| marge - teinte - pigmentation | TDA - | 8 | |
| plate, marge irrégulière, trans- | IND - | 9 | |
| lucide,circulaire, | VP + | 10 | |
| * état frais : forme - mobilité | GEL - | 11 | |
| bacille, mobile | GLU - | 12 | |
| * Gram - spore | MAN - | 13 | |
| GRAM - | INO - | 14 | |
| PHYSIOLOGIE : | SOR - | 15 | |
| | RHA - | 16 | |
| * type respiratoire:aérobie strict | SAC - | 17 | |
| * oxydase: + | MEL - | 18 | |
| * catalase: + | AMY - | 19 | |
| * nitrate réductase: NO3 | ARA - | 20 | |
| * type métabolique: oxydatif | | | |

-----

CULTURE SUR POLLUANT

c: +/-

SOUCHE :

XInb5

ORIGINE :
Site contaminé par les PCBs

MORPHOLOGIE :

* colonie : forme - hauteur -
  marge - teinte - pigmentation
légèrement bombée, marge régulière
crème, circulaire,
* état frais : forme - mobilité
bacille, non mobile
* Gram - spore
GRAM -

PHYSIOLOGIE :

* type respiratoire:aérobie strict
* oxydase: +
* catalase: +
* nitrate réductase: NO2
* type métabolique: alcalinisante

| | API 20E | APIZYM |
|---|---|---|
| ONPG- | 1 | |
| ADH - | 2 | 1 |
| LDC - | 3 | 1 |
| ODC - | 4 | 2 |
| CIT +/- | 5 | |
| H2S - | 6 | 1 |
| URE - | 7 | |
| TDA - | 8 | |
| IND - | 9 | |
| VP + | 10 | |
| GEL - | 11 | |
| GLU - | 12 | |
| MAN - | 13 | |
| INO - | 14 | |
| SOR - | 15 | |
| RHA - | 16 | |
| SAC - | 17 | |
| MEL - | 18 | |
| AMY - | 19 | |
| ARA - | 20 | |

CULTURE SUR POLLUANT

bp: ++
c:  -

**IV - EXEMPLE DE DECONTAMINATION D'UNE TERRE CONTENANT DE L'AROCLOR 1260**

Il s'agit de terre obtenue suite à un accident de transformateur en région parisienne. La contamination initiale fait l'objet d'un examen approfondi.

La teneur en argile est de 14,5%.

## IV TENEUR INITIALE ET PLAN D'ETUDES

4 à 5 tonnes de terre sont disposées en tas. 5 tas de 50 à 60 Kg sont individualisés. Leur point d'origine est dispersé par rapport au tas initial, mais déterminé au hasard.

Chacun de ces 5 prélèvements étant destiné à un essai spécifique, nous le repérons selon le nom donné aux essais.

Dans chaque prélèvement, 10 sous-échantillons sont réalisés au hasard, puis analysés. Les résultats sont présentés dans le tableau suivant :

| ESSAIS | CL | C1 | C8 | C9 |
|---|---|---|---|---|
| ANALYSES (ppm) | 100 | 102 | 87 | 103 |
| | 79 | 108 | 130 | 147 |
| | 78 | 93 | 138 | 173 |
| | 90 | 80 | 132 | 89 |
| | 73 | 124 | 114 | 104 |
| | 150 | 99 | 90 | 108 |
| | 75 | 121 | 240 | 103 |
| | 68 | 78 | 109 | 95 |
| | 74 | 113 | 113 | 145 |
| | 97 | 87 | 110 | 119 |
| MOYENNES | 88 | 101 | 126 | 119 |
| ECART-TYPE | 24 | 16 | 43 | 27 |

La teneur moyenne dans ce sol est de 115 ppm, mais avec un écart-type élevé de 33. Par conséquent, il y a une très forte probabilité pour qu'un échantillon prélevé au hasard ait une teneur comprise entre 82 et 148 ppm. Les minima et maxima sont respectivement 68 et 240 ppm. Par essai, la moyenne et son écart-type, les minima et maxima sont connus.

Plan d'études :

CL : caisson L, apports volontaires de minéraux et avec l'inoculum IV

CL : caisson 1, inoculum XI et apport de tensioactif

C8 : caisson 8, inoculum IV

C9 : caisson 9, inoculum XI

Le tableau suivant présente l'ensemble des apports d'inoculum réalisés, sur les caissons :

| TEMPS | CAISSONS | | | |
|---|---|---|---|---|
| | CL | C1 | C8 | C9 |
| to | + | + | + | + |
| to + 0,5 m | + | + | + | + |
| to + 1 m | + | + | + | + |
| to + 2,3 m | + | + | + | + |

+ : apport de 500 ml d'inoculum, sauf à to + 2,3 mois où l'apport est de :

      1000 ml pour C1 et CL,

       500 ml pour C8,

       350 Ml pour C9.

**IV.2 RESULTATS**

Le tableau suivant présente l'ensemble des résultats obtenus (ppm) :

| TEMPS | CL | C1 | C8 | C9 |
|---|---|---|---|---|
| to + 1 m | 140 | 99 | 157 | 119 |
| to + 1,25 m | – | 70 | – | – |
| to + 2,25 m | 70 | 85 | 139 | 124 |
| | 105* | 75* | | |
| to + 4 m | 76 | 100 | 106 | 126 |
| to + 4,5 m | – | 79 | – | – |
| to + 5,5 m | – | 62 | – | – |
| to + 6 m | – | – | 88 | 107 |

\* : autre prélèvement et laboratoire d'analyse.

Tous les prélèvements sont réalisés au niveau 1 et en zone rampe.

C1 connait une baisse de teneur d'environ 33%.

C8 connait une baisse de teneur d'environ 25%.

**IV.4 CONCLUSION**

Le caisson 1 (apport de tensioactif) appparait comme le plus efficace.

**IV.4 REMARQUES**

a\ présence de corps en suspension dans l'eau de process :
– C1, corps provenant du bac d'eau, présentant une teneur de 99 ppm;

– CL, teneur en PCBs dans l'eau de process, débarrassée des corps en suspension, à to + 2,25 mois : 1 ppb.

b\ débit de l'eau de recirculation :

environ 200 ml/heure

c\ apport total en minéraux

| CAISSONS | CL | C1 | C8 | C9 |
|---|---|---|---|---|
| Vol. Tot. d'Inoculum | 2,5 | 2,5 | 2,0 | 1,85 |
| Apport compl. | 2 | 0 | 0 | 0 |
| Minéraux (g) : | | | | |
| N | 4,17 | 1,38 | 0,66 | 1,10 |
| P | 1,71 | 2,87 | 0,45 | 2,29 |
| K | 2,80 | 6,08 | 1,10 | 4,86 |
| Fe | 0,03 | <1mg | <1mg | <1mg |
| Zn | 0,02 | 0 | <1mg | 0 |
| Cu | 0,02 | 0 | <1mg | 0 |
| Mg | 1,09 | <1mg | 0,18 | <1mg |
| S | 0,30 | 0,008 | 0,24 | 0,006 |
| Na | 7,03 | 0 | 1,08 | 0 |
| Mn | 0 | <1mg | 0 | <1mg |
| Ca | 0 | <1mg | 0 | <1mg |

## V - EXEMPLE DE DECONTAMINATION D'UNE TERRE CONTENANT DE L'AROCLOR 1242

La terre initiale est appelée Terre 3. Il s'agit du seul type disponible le jour de la contamination volontaire. Sa teneur en argile est de 13,2%.

La contamination par l'Aroclor 1242 est réalisée avec un pulvérisateur qui projette le pyralène sur la terre, sous la forme d'un brouillard fin. Cependant la répartition reste manuelle et le temps nécessaire pour la contamination de 50 litres de terre est d'environ 30 mn.

Après contamination 6 échantillons sont prélevés.

Résultats :

| Teneurs | 122  195  154  93  279  179 |
|---|---|

Moyenne (M) :     170

Ecart-type (s) :     65

Le caisson est inoculé avec 700 ml d'inoculum à to (Temps initial).Cet inoculum correspond à l'inoculum IV de l'exemple précédent et contient des Fusarium et des levures.

D'autres apports d'inoculum sont effectués par la suite, après prélèvement de sol pour analyse :

| Temps | Volume apporté (ml) |
|---|---|
| to+ 1,5 mois | 500 |
| to+ 2,0 mois | 500 |
| to+ 2,5 mois | 500 |
| to+ 3,5 mois | 500 |
| to+ 5,5 mois | 1000 |
| to+ 7,0 mois | 500 |
| to+ 8,0 mois | 1000 |
| to+ 9,0 mois | 1000 |

**V.1 SUIVI ANALYTIQUE**

Résultats :

| Temps | Teneurs (ppm) | M |
|-------|---------------|---|
| to+ 1,5 mois | 75 | 84 |
|  | 92 |  |
| to+ 2,0 mois | 69 | 73 |
|  | 76 |  |
| to+ 2,5 mois | 92 | 81 |
|  | 70 |  |
| to+ 3,0 mois | 66 |  |
|  | 87 | 88 |
|  | 112 |  |
| to+ 3,5 mois | 48 |  |
| to+ 5,5 mois | 70 |  |
|  | 72 |  |
|  |  | 66 |
|  | 58 |  |
|  | 64 |  |
| to+ 7,0 mois | 39 | - |
| to+ 8,0 mois | 32 | - |
| to+ 9,0 mois | 32 | - |

M : moyenne

A to+ 1,5 mois, une différence de 50% est observée; elle reste globalement stable jusqu'à to+ 3 mois. Ces résultats, sont à nuancer en fonction de

l'hétérogénéité initiale. En effet l'écart-type sur les valeurs obtenues entre to+ 1,5 mois et to+ 3 mois (inclus) n'est que de 15, alors que sur la terre initiale il est de 65.

A to+ 3,5 mois, un écart significatif est relevé par rapport à la teneur initiale, qui se confirme par la suite. Les résultats obtenus à to+ 8 et to+ 9 mois indiquent un abattement de 80% environ.

## VI - ANALYSE ULTRASTRUCTURALE

Dans un premier temps, les analyses ultrastructurales concernent les expérimentations sur traitement de sols contaminés par l'Aroclor 1260. Deux inoculums sont utilisés : XI et IV.

Les échantillons correspondent à des masses visqueuses observées en tous lieux du circuit eau, que ce soit avec les réacteurs de 6 litres ou les caissons.

1. le point d'écoulement de l'eau lixiviée à la base d'un réacteur de 6 litres, où l'inoculum IV est utilisé,

2. une tuyauterie de lixiviation où l'inoculum XI est utilisé sur un caisson,

3. au même niveau que 2, sur un caisson utilisant l'inoculum IV,

4. dans le bac d'eau aéré d'un caisson différent de 2, où il s'agit encore de l'inoculum XI.

Dans un second temps, les mêmes analyses sont effectuées sur des cultures en erlen. La composition minérale est propre au type de souches, eucaryotes ou procaryotes, et reste similaire à celle présentée pour la préparation d'inoculum. Les cultures sont obtenues sous un mouvement vortex ou orbital. Elles sont récupérées par centrifugation (5000 tours/minutes) à une température comprise entre 1 et 16°C. Elles proviennent de différentes sources de carbone, soit en fonction de l'échantillon :

5. Témoin A. Inoculum XI développé en présence de 10 grammes de saccharose/litre. Le temps de culture est de 2 jours.

6. Témoin B. Inoculum IV développé en présence de 10 grammes de saccharose/litre. Le temps de culture est de 4 jours.

7. Inoculum XI développé en présence de 1,5 g de saccharose et de 1 ml d'Aroclor 1260/litre. Le temps de culture est de 15 jours, sous mouvement vortex.

8. Inoculum IV développé dans les mêmes conditions que 7.

9. Inoculum XI développé dans les mêmes conditions que 7, sauf que le temps de culture est de 30 jours.

10. Inoculum IV développé dans les mêmes conditions que 8, sauf que le temps de culture est de 30 jours.

11. Inoculum XI développé dans les mêmes conditions que 7, sauf que le mouvement est de type orbital.

12. Inoculum IV développé dans les mêmes conditions que 8, sauf que le mouvement est de type orbital.

Les techniques de fixation et traitement sont propres à chaque type d'observation.

### VI.1 Observations au microscope électronique à transmission (M.E.T.)

Cette technique est utilisée pour l'observation de coupes fines en vue d'observer les détails internes des cellules.

Préparation du matériel biologique :

- Fixation

Les échantillons sont placés dans un tube contenant un mélange de glutaraldéhyde à 2,5% et de tampon phosphate M/10 à pH 7,2 pendant 6 à 8 heures à la température de la glace fondante. Les objets sont ensuite rincés avec le même tampon et laissés toute une nuit.

Le lendemain, ils sont postfixés à l'osmium à 2% dans le tampon phosphate pendant 1 heure.

- Déshydratation

Après la postfixation osmique, les objets sont rincés plusieurs fois avec de l'eau distillée puis déshydratés par des bains d'alcool de concentration croissante :

10% pendant 1/4 d'heure,
20% pendant 1/4 d'heure,
40% pendant 1/4 d'heure,
60% pendant 1/4 d'heure ou toute une nuit,
80% pendant 1/4 d'heure,
95% deux fois 20 minutes,
100% trois fois 20 minutes.

- Imprégnation et inclusion

Les objets sont progessivement imprégnés par la résine (EPON 812) :
– mélange 2/3 acétone et 1/3 résine durant une journée,
– mélange 1/3 acétone et 2/3 résine durant une seconde journée.
Ils sont ensuite placés dans des moules remplis de résine pure et mis à l'étuve (55°C) pendant 24 à 48 heures.

- Coupe et contraste

Les coupes fines (800 à 1000 Å) sont réalisées à l'aide d'un ultramicrotome équipé d'un couteau diamant. Elles sont recueillies sur des grilles en cuivre (300 et 200 mesh) puis colorées pendant 10 minutes par le citrate de plomb (REYNOLDS E.S. (1963) Journal cell. Biology, Vol 17:208-212). Les grilles sont soigneusement rincées à l'eau distillée et peuvent alors être observées au microscope électronique SIEMENS ELMISKOP. Des coupes sont aussi recueillies sur des grilles en or pour pratiquer des tests cytologiques.

- Test cytologiques

Le test PATAG est uniquement utilisé pour la recherche des polysaccharides (THIERY J.P. (1967) Journal microscopie, Vol. 6:987-1018).
Les objets subissent une oxydation par l'acide périodique à 1% pendant 30 minutes, puis avec un rinçage soigneux avec l'eau distillée, les grilles sont immergées dans une solution de thiocarbohydrazide à 0,2 % dans l'acide acétique à 20% pendant 5 à 7 heures (température ambiante).
Les grilles sont ensuite lavées dans des solutions d'acide acétique à concentration décroissante (20, 15, 10 et 5%) à raison de deux bains de 5 minutes pour chacune d'elles.
Après un rinçage très soigneux avec l'eau distillée, les grilles sont immergées dans une solution de protéinate d'argent à 1 % dans l'eau bidistillée durant 30 minutes à l'obscurité et à température ambiante.
Les grilles sont ensuite rincées plusieurs fois à l'eau bidistillée et peuvent être observées au microscope électronique.

## VI.2 Observation au microscope électronique à balayage (M.E.B.)

Cette technique est intéressante lorsqu'il s'agit d'observer la surface de l'échantillon. Le faisceau électronique est focalisé sur l'objet. La préparation du matériel biologique reste semblable à celle concernant les observations pour le M.E.T., en ce qui concerne le prélèvement et la fixation.
Par contre, la déshydratation est réalisée avec des bains d'acétone à concentration croissante.
Une nouvelle phase intervient alors, le séchage de l'échantillon réalisé par le technique du "Point Critique". Celle-ci consiste à substituer dans un premier temps l'acétone par du $CO_2$ liquide, puis dans un second temps à éliminer ce dernier par chauffage pour ne conserver que l'échantillon sec.
L'échantillon sec est ensuite métallisé à l'or avant observation. Elle est effectuée sur un microscope électronique SIEMENS AUTOSCAN.
L'inoculum XI concentre de façon remarquable les granules (photo 1). Ils sont présents dans la structure fibrillaire et granuleuse. Ces "exsudats" ne sont pas de nature polyosidique. Ils proviennent sans doute de la lyse cellulaire induite par la présence de l'Aroclor 1260. Lors de la mise en évidence des polyosides, les granules et la stucture fibrillaire et granuleuse ne sont pas concernés.
Comme le contraste au citrate de plomb n'a pas d'effet sur la densité électronique des granules, il est admis que ceux-ci n'ont pas de relations étroites avec des protéines de l'autolyse bactérienne. L'absence de granules lors du contraste des polyosides montre que le traitement les extrait. Il semble exister une relation entre les protéines de lyse bactérienne et les granules. Cette lyse est évidemment provoquée par l'Aroclor 1260.

Les granules se rencontrent dans les cellules de bactéries mortes à structure partiellement désorganisée et dans les cellules des bactéries vivantes.

La morphologie initiale des bactéries est modifiée en présence de l'Aroclor 1260 ce qui se traduit par un allongement inhabituel des cellules, une désorganisation ultrastructurale et une augmentation de la zone externe.

Sous mouvement orbital, les bactéries s'accumulent sous forme de boules, ce qui empêche la pénétration des granules à l'intérieur de ces agrégats. Il y a donc une perte de pouvoir récupérateur. La production d'inoculum bactérien en mouvement orbital est en conséquence déconseillée.

L'inoculum IV ne présente une incorporation des granules dans la structure pariétale des champignons et des levures que dans certains cas. Ces cellules ne possèdent pas de zone filamenteuse et granuleuse semblable à celle des bactéries. Ainsi, il apparaît que cet inoculum semble moins efficace vis-à-vis de l'accumulation des granules denses.

**Dosage des PCBs**

1) Les dosages sont effectués sur des extraits hexaniques préparés sous ébullition pour les sols et solides.

- Principe :

   * extraction à l'hexane des PCBs contaminant un sol,
   * dosage par chromatographie en phase gazeuse (CPG) de l'extrait par comparaison avec des solutions d'un étalon d'Aroclor.

- Réactifs :

   Hexane PESTIPUR 99 (Société S.D.S.)
   Acide sulfurique concentré
   Gammes étalon en solution hexanique d'Aroclor 1242 et 1260
   (3 dilutions au maximum comprises entre 50 ppb et 1 ppm)

- Appareillages :

   . Soxhlet (ballon de 500 ml),
   . étuve à 50°C,
   . capsules en porcelaine,
   . mortier en porcelaine,
   . chromatographe en phase gazeuse (CPG) VARIAN 3300, équipé d'un détecteur à capture d'électrons, d'un injecteur split-splitless et d'une colonne DB5 (J. et W. Scientific) présentant une longueur de 30 m et une épaisseur de phase de 0,25μ. Le gaz vecteur est l'azote B50 N50 (Alphagaz).

Tout le matèriel utilisé est soigneusement lavé avec une solution de sulfochromique avant d'être rincé abondamment à l'eau, puis à l'acétone.

- Extraction des PCBs contaminant un sol :

   . séchage du substrat à 50°C pendant 48 heures en minimum, jusqu'à poids constant,
   . broyage dans un mortier,
   . pesée de 20 g de substrat sec (M),
   . ébullition de 200 ml d'hexane (V3) en présence du substrat sec, sous reflux pendant 6 heures,
   . après refroidissement, le ballon étant incliné, la phase hexanique exempte de particules est récupérée.

- Chromatographie :

   . Conditions opératoires :
   températures                        : Injecteur, 250°C
                                          Colonne, gradient de 4°C/mn, de 190 à 250°C isotherme pendant 6 minutes

Détecteur, 300°C

split ouvert : débit 25 ml/mn

make up : débit 30 ml/mn

pression entrée colonne : 13 psi

– La ou les gammes étalon (Aroclor 42 et Aroclor 60) sont réalisées chaque jour avec trois dilutions différentes au minimum.

– Injection de 2µl d'extrait, convenablement dilué afin de se situer dans la gamme de mesure.

– Un extrait trop dilué est concentré au moyen de l'évaporateur rotatif.

2) <u>Expression des teneurs</u>

Sur le chromatogramme des étalons et extraits, 15 à 20 pics parmi les plus importants quantitativement,

- calcul de la somme des aires de ces pics :

soit :

So, somme des pics de la solution de l'étalon la plus proche de l'extrait à doser,

S1, somme de ces mêmes pics dans l'extrait,

Co, concentration en PCBs de l'étalon (mg/l ou ppm),

C1, concentration en PCBs dans l'extrait.

$$C1 = Co \times \frac{S1}{S0}.$$

Concentration en PCBs dans la terre (C') :

$$C' = C1 \times \frac{V3}{M} = Co \times \frac{S1}{S0} \times \frac{V3}{M}$$

où,

V3 correspond au volume d'extrait,

M correspond à la masse de substrat sec.

L'incertitude relative est d'environ 12%.

**Revendications**

1/ Composition caractérisée en ce qu'elle est destinée à la décontamination d'un sol pollué par un ou plusieurs contaminants toxiques déterminés, et en ce qu'elle comprend un inoculum à base de cellules procaryotes et/ou eucaryotes notamment de bactéries, de champignons ou de levures ou plusieurs types de ces cellules, sélectionnées pour leur capacité à :

– se développer dans un milieu de culture contenant comme seule source de carbone des contaminants toxiques déterminés, en particulier des contaminants organiques, et

– capter, adsorber et accumuler ces contaminants à partir d'un sol à traiter, au niveau de leur paroi cellulaire et éventuellement au niveau de leur cytoplasme et/ou par l'intermédiaire de leurs produits de lyse.

2/ Composition selon la revendication 1, caractérisée en ce qu'elle est sensiblement dépourvue de cellules ne se développant pas en présence d'une source de carbone constituée exclusivement par les susdits contaminants toxiques.

3/ Composition caractérisée par les propriétés suivantes :

– elle est destinée à la décontamination d'un sol pollué par un ou plusieurs contaminants toxiques déterminés,

– elle est obtenue à partir des souches cellulaires naturellement présentes dans un sol contaminé, par sélection par exemple en mettant en oeuvre les étapes suivantes :

a) l'ensemencement d'un milieu de culture minéral adapté pour le développement d'organismes cellulaires du type procaryotes et/ou eucaryotes, ce milieu contenant en outre à titre de seule source de carbone, au moins un contaminant toxique déterminé, avec une quantité déterminée de cellules eucaryotes ou procaryotes choisies,

b) l'incubation à une température donnée, de préférence la température optimale de croissance des souches eucaryotes ou procaryotes par exemple entre 4°C et 35°C environ de préférence entre 10°C et 35°C, notamment supérieure à 15°C, de façon particulièrement avantageuse entre environ 20 et environ 32°C, de façon particulièrement avantageuse environ 30°C pour des bactéries et environ 25°C pour des microorganismes filamenteux ou des levures pendant un temps suffisant pour favoriser le développement des cellules résistantes aux contaminants,

c) l'étalement sur un milieu nutritif spécifique des différents types cellulaires présents pour estimer leur capacité de développement,

d) le cas échéant un ou plusieurs repiquages successifs des cultures avec des concentrations croissantes de contaminants et la répétition de l'étape c) après chaque repiquage,

e) la culture avec un milieu nutritif exempt d'extrait de levure, spécifique des différents types cellulaires présents pour estimer leur capacité de développement,

f) la récupération des souches capables de se développer dans ce milieu.

4/ Composition selon la revendication 3, caractérisée en ce qu'elle est obtenue après sélection des souches récupérées capables de se développer dans un milieu déterminé, cette sélection comprenant l'observation au microscope - électronique à transmission de l'incorporation par les cellules, des contaminants toxiques et la récupération de ces cellules.

5/ Composition selon l'une quelconque des revendications 1 à 4, caractérisée en ce que l'inoculum comprend des cellules capables de se développer en culture avec des contaminants organiques de la famille des PCBs, en particulier avec des PCBs hautement substitués, ces cellules étant en outre capables de les capter, de les adsorber, et de les accumuler au niveau de leur paroi cellulaire, voire au niveau de leur cytoplasme et/ou par l'intermédiaire de leurs produits de lyse.

6/ Composition selon l'une quelconque des revendications 1 à 5, caractérisée en ce que l'inoculum comprend des cellules capables de se développer en culture avec des contaminants organiques toxiques différents des PCBs, halogénés ou non halogénés, possédant le cas échéant un ou plusieurs cycles aromatiques, notamment des huiles lourdes, des pesticides, des fongicides, des insecticides ou des solvants organiques, ou encore avec des métaux lourds, les cellules de l'inoculum étant en outre capables de capter, d'adsorber, et d'accumuler ces contaminants organiques toxiques au niveau de leur paroi cellulaire.

7/ Composition selon l'une quelconque des revendications 1 à 6, caractérisée en ce que l'inoculum contient des cellules capables en outre de métaboliser, au moins partiellement, les contaminants organiques toxiques.

8/ Composition selon l'une des revendications 1 à 5, caractérisée en ce que l'inoculum comprend des cellules permettant ou favorisant la désorption des contaminants toxiques associés à certaines fractions du sol.

9/ Composition selon l'une quelconque des revendications 1 à 8, caractérisée en ce que l'inoculum est constitué de cellules vivantes, de même origine ou non, ou d'un mélange de cellules vivantes et de cellules mortes, de même origine ou non, ayant conservé leur capacité de captation, d'adsorption et d'accumulation.

10/ Composition selon l'une quelconque des revendications 1 à 9 , caractérisée en ce l'inoculum contient des des champignons notamment filamenteux en particulier de la famille des Ascomycètes, par exemple Fusarium, Aspergillus, ou Penicillium et/ou d'autres cellules eucaryotes par exemple des levures.

11/ Composition selon l'une quelconque des revendications 1 à 5, caractérisée en ce que l'inoculum contient une ou plusieurs types de cellules eucaryotes et/ou un ou plusieurs types de cellules procaryotes parmi XIbp1, XIbp3, XIbp4, XIbp6, XInb2, XInb5, XIc1 ou encore l'ensemble de ces cellules en mélange.

12/ Composition selon l'une quelconque des revendications 1 à 11, caractérisée en qu'elle contient en outre des nutriments capables de stimuler la croissance des cellules de l'inoculum et/ou leur activité dans le sol.

13/ Composition selon l'une quelconque des revendications 1 à 12, caractérisée en ce qu'elle contient en outre un agent tensio-actif, ou une souche cellulaire capable de produire un agent tensio-actif.

14/ Composition selon l'une quelconque des revendications 1 à 13, caractérisée en ce que les parois des cellules de l'inoculum sont modifiées de façon à augmenter leur capacité d'adsorption des contaminants toxiques, la modification résultant par exemple de l'accroissement de leur épaisseur ou de l'épaisseur de la zone mucilagineuse externe, sous l'effet de lyse des produits toxiques, ou de l'augmentation de la surface de ces parois .

15/ Composition selon l'une quelconque des revendications 1 à 14, caractérisée en ce qu'elle comprend au moins un prédateur des cellules de l'inoculum, notamment une amibe, par exemple T Graniféra.

16/ Composition selon l'une quelconque des revendications 1 à 15, caractérisée en ce qu'il s'agit soit d'une solution comprenant l'inoculum sous forme de cellules en suspension, soit d'une composition lyophilisée.

17/ Utilisation d'une composition selon l'une quelconque des revendications 1 à 16, pour le traitement de sols contaminés par des contaminants toxiques, en particulier des contaminants organiques toxiques.

18/ Procédé de traitement de sols contaminés par des contaminants toxiques en particulier des contaminants organiques, caractérisé en ce qu'il comprend l'administration à travers une quantité donnée de sol d'une composition selon l'une quelconque des revendications 1 à 16, dans des conditions permettant la lixiviation de cette composition à travers le sol et, la récupération dans l'eau de lixiviation des cellules ayant accumulé les contaminants toxiques.

19/ Procédé de traitement selon la revendication 15, caractérisé en ce qu'il comprend :

– l'épandage et/ou l'injection dans le sol d'une composition selon l'une quelconque des revendications 1 à 16 de façon à permettre la lixiviation de cette composition à travers le sol, dans des conditions telles que les contaminants toxiques du sol sont captés par les cellules de l'inoculum, adsorbés au niveau de leur paroi cellulaire et éventuellement de leur cytoplasme, ou encore dans des conditions telles que les contaminants toxiques sont captés par l'intermédiaire des produits de lyse des cellules de l'inoculum, ces contaminants étant le cas échéant métabolisés, et l'inoculum agissant éventuellement après désorption à partir de certaines fractions du sol,

– la récupération de l'eau de lixiviation contenant notamment les cellules ayant adsorbé les contaminants toxiques,

– le traitement de l'eau de lixiviation pour séparer les cellules ayant accumulé les contaminants toxiques,

– le cas échéant la répétition des étapes précédentes, à partir de l'eau de lixiviation traitée.

20/ Procédé selon l'une quelconque des revendications 18 ou 19, caractérisé en ce que plusieurs administrations de l'inoculum sont effectuées au cours du traitement, par exemple au stade de l'administration à travers le sol et au stade de la récupération de l'eau de lixiviation.

21/ Procédé selon l'une quelconque des revendications 18 ou 19, caractérisé en ce que l'inoculum administré contient des cellules mortes, ayant conservé leur capacité de captation, d'adsorption et d'accumulation.

22/ Procédé selon l'une quelconque des revendications 18 à 21, caractérisé en ce que l'on épand avec la composition selon l'une quelconque des revendications 1 à 13, des composés permettant d'augmenter l'épaisseur de la paroi et/ou l'épaisseur de la zone mucilagineuse externes des cellules de l'inoculum et/ou des microorganismes dont la lyse libère des produits captants.

23/ Procédé selon l'une quelconque des revendications 18 à 22, caractérisé en ce que la récupération des contaminants toxiques accumulés par les cellules de l'inoculum est réalisée par floculation du matériel cellulaire présent dans l'eau de lixiviation et/ou par coagulation des débris cellulaires ou encore par dégradation des cellules par un prédateur, par exemple une amibe, par exemple T Granifera, dégradation suivie d'une récupération des PCBs.

24/ Dispositif pour mettre en oeuvre le procédé selon l'une quelconque des revendications 18 à 23, caractérisé en ce qu'il comprend :

– des moyens pour réaliser l'épandage et/ou l'injection dans le sol d'une composition selon l'une quelconque des revendications 1 à 16, et permettre la lixiviation de cette solution à travers la terre à traiter,

– des moyens pour recueillir et le cas échéant traiter l'eau de lixiviation.

M . E . T .

1 cm ---> 0,16 µ

FIGURE 1

M . E . T .

1 cm ---> 3 µ

FIGURE 2

M . E . T .

1 cm ——> 0,22 μ

FIGURE 3

M.E.T.

FIGURE 4

1 cm ——> 0,4 μ

M . E . T .

1 cm ——> 0,3 μ

FIGURE 5

M . E . T .

1 cm ——> 0,4 µ

FIGURE 6

Etapes pour l'obtention de souches sélectionnées

SUSPENSION-
DILUTION

ETAPE 1

Incubation

Etalement sur milieu gélosé (e)

ETAPE 4

Incubation          Incubation          ETAPE 2

(e)

ETAPE 3          (e)

Etalement en milieu gélosé + contaminant

ETAPE 5 ⟶ Incubation ⟶ SOUCHES SELECTIONNEES

FIGURE 7

**Office européen des brevets**

# RAPPORT DE RECHERCHE EUROPEENNE

Numero de la demande

EP    91 40 3529

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 554 075 (HOU-MIN CHANG)<br>* revendications *<br>--- | 1 | B09B3/00<br>C12N1/20<br>C12N1/14<br>//(C12N1/20;<br>C12R1:00)<br>(C12N1/14;<br>C12R1:645) |
| X | WO-A-9 010 079 (ALKO)<br>* revendications *<br>--- | 1 | |
| X | US-A-3 979 283 (CAROLINA M. PRUDOM)<br>* revendications *<br>--- | 1 | |
| X | GB-A-2 010 327 (BAYER)<br>* revendications *<br>--- | 1 | |
| X | US-A-4 843 009 (LAWRENCE H. BOPP)<br>* revendications *<br>--- | 1 | |
| X | DERWENT WPIL<br>DERWENT PUBLICATIONS LONDON<br>AN 70-40933R<br>& SU-A-250850 (MICROBIOLOGY INSTITUTE)<br>--- | 1 | |
| Y | DE-A-3 537 307 (NORDDEUTSCHE SEEKABELWERKE)<br>* revendications *<br>--- | 1 | **DOMAINES TECHNIQUES RECHERCHES (Int. Cl.5)** |
| Y | WATER RESEARCH.<br>vol. 23, no. 5, Mai 1989, OXFORD GB<br>pages 561 - 568;<br>M. TSEZOS ET AL.: 'Comparison of the biosorption and desorption of hazardous organic'<br>* le document en entier *<br><br>----- | 1 | B09B<br>CO2F<br>C12N<br>C12R<br>A62D |

Le présent rapport a été établi pour toutes les revendications

| Lieu de la recherche | Date d'achèvement de la recherche | Examinateur |
|---|---|---|
| LA HAYE | 07 AVRIL 1992 | DELANGHE L.L.M. |

EPO FORM 1503 03.82 (P0402)